# EUROPEAN PATENT SPECIFICATION

(11) **EP 1 210 936 B1**
(45) Date of publication and mention of the grant of the patent: **24.09.2003**
(21) Application number: 01300216.7
(22) Date of filing: 11.01.2001
(51) Int. Cl.: A61K 9/48

(54) **Gelatin capsule**
Gelatinkapsel
Capsule de gélatine

(30) Priority: 28.11.2000 KR 2000071155
(43) Date of publication of application: 05.06.2002
(73) Proprietor: Suheung Capsule Co Ltd., Bucheon, Kyonggi-do 422-040 (KR)
(72) Inventor: Yang, Joo Hwan, Bucheon, Kyonggi-do (KR)
(74) Representative: Cockbain, Julian, Dr.

(56) References cited:
- EP-A- 0 600 422
- DATABASE CA [Online] CHEMICAL ABSTRACTS SERVICE, COLUMBUS, OHIO, US; YAMADA, TATSU ET AL: "Glycerol diacetate as adhesion inhibitor in manufacturing pharmaceutical soft capsules by molding apparatus" retrieved from STN Database accession no. 1993:198197 XP002194774 & JP 04 288012 A (TOKAI CAPSULE CO., LTD., JAPAN) 13 October 1992 (1992-10-13) -& DATABASE WPI Section Ch, Week 199247 Derwent Publications Ltd., London, GB; Class B07, AN 1992-387686 XP002195666 & JP 04 288012 A (TOKAI CAPSULE CO., LTD., JAPAN), 13 October 1992 (1992-10-13)
- KIBBE AH (ED.): "Handbook of pharmaceutical excipients" 20 September 2000 (2000-09-20) , PHARMACEUTICAL PRESS , LONDON XP002194773 Sodium Lauryl Sulfate, page 487 - page 489 * page 487, line 25 *

## Description

The present invention relates to gelatin capsules and their preparation.

Gelatin capsules, in particular so-called hard or hardshell gelatin capsules, are widely used to contain pharmaceuticals, vitamins, essential oils, neutraceuticals, etc. and are normally prepared in two component parts, a body and a cap, which are joined together to produce the final closed capsule.

Gelatin capsules have conventionally been manufactured by a process comprising the following steps:
i) dipping a mold pin in a gelatin solution in a capsule manufacturing machine so as to mold and dry a gelatin film,
ii) stripping off the molded gelatin film from the mold pin and trimming it;
iii) combining capsule caps and bodies to form capsules, e.g. at a metallic joiner block; and
iv) ejecting the prepared gelatin capsule, e.g. using an ejector rod.

In the course of joining the cap to the body, static electricity is sometimes generated which induces low film flexibility, which causes the cap and body to telescope or otherwise join badly, a critical failure in capsule manufacturing. A bad joint between cap and body may cause these to separate during transportation, it may cause mistakes (e.g. in alignment) during capsule imprinting processes, and it may cause mistakes to occur in the filling of the capsule.

To solve the above problems, it has been conventional to add glycerine or sorbitol to the gelatin solution as a plasticizer to maintain the flexibility of the capsule component films. However, such plasticizers slow the drying of the film after the molding step in the capsule manufacturing machine. Further, they also cause problems such as deformation and contraction of the cap or body during long term storage. Therefore, such plasticizers increase the instability of gelatin capsules with time.

We have now found that these problems may be ameliorated or avoided by the inclusion of a diacetylated monoglyceride and a surfactant, e.g. an anionic surfactant, for example an alkali or alkaline earth metal lauryl sulphate, such as sodium lauryl sulphate, which together serve to reduce the static electricity and enhance surface lubrication of the gelatin films.

In Japanese laid open patent No. 6-157916, it was proposed that 3-10 wt% of glycerine fatty acid ester be added to the gelatin solution during the manufacture of the gelatin capsule film. Further, as an example of glycerine fatty acid esters, diacetylated monoglycerides were disclosed. However, the declared purpose for using the glycerine fatty acid ester was to maintain the elasticity of the gelatin film at the time of filling a hygroscopic drug into the capsule so as to protect against absorption of moisture by the drug from the gelatin capsule film. Moreover the ester was intended to such protect against leakage of the drug in the event of external impact on the capsule.

Thus viewed from one aspect the invention provides a process for the preparation of gelatin capsules, in particular capsules having reduced incidence of static electricity and enhanced capsule film surface lubrication, especially so-called hard shell gelatin capsules, said process comprising:
i) forming an emulsion, preferably an aqueous emulsion, particularly an oil-in-water emulsion, containing diacetylated monoglyceride and sodium lauryl sulphate;
ii) mixing said emulsion and a gelatin solution, preferably an aqueous solution, in a weight ratio of 0.3 to 1.0 parts diacetylated monoglyceride and surfactant to 100 parts gelatin; and
iii) forming said capsules.

Diacetylated monoglycerides and surfactants such as sodium lauryl sulfate are referred to in the US National Formulary and elsewhere in the chemical literature. In the US National Formulary, diacetylated monoglycerides are described as having various usages, for example as anti-dusting agents, deforming agents, lubricants, stabilizers, plasticizers and release agents.

In a preferred embodiment, the process of the invention comprises the steps of:
i) preparing an emulsion containing 0.3-1.0 parts by weight of diacetylated monoglycerides and sodium lauryl sulfate;
ii) adding said emulsion to a gelatin solution containing 100 parts by weight of gelatin to form a mixed solution;
iii) mixing and homogenizing said mixed solution; and
iv) adjusting viscosity, standing for a foam-off period, and forming a capsule.

Viewed from a further aspect the invention provides the use of a diacetylated monoglyceride and a surfactant, especially an anionic surfactant, particularly sodium lauryl sulfate, in the manufacture of gelatin capsules and gelatin capsule components.

Viewed from another aspect the invention provides a gelatin capsule, the gelatin shell whereof comprises diacetylated monoglyceride, surfactant (especially an anionic surfactant such as sodium lauryl sulfate) and gelatin in a weight ratio of diacetylated monoglyceride and surfactant to gelatin of 0.3:100 to 1.0:100.

Viewed from a still further aspect the invention provides gelatin capsules and capsule components prepared by the process of the invention.

The diacetylated monoglycerides used in the process of the invention are preferably liquid (more especially clear liquids) at ambient temperature (e.g. 21°C) and typically will include a C₁₂ to C₂₅, more particularly C₁₄ to C₂₀, especially C₁₆ to C₁₈, fatty acid residue (e.g. RCO where R is an alkyl or singly or multiply unsaturated alkenyl group, particular an alkyl or singly or double unsaturated alkenyl group). The diacetylated monoglycerides are available commercially or may be prepared by conventional synthetic techniques. The diacetylated monoglyceride is preferably used in a weight range of 0.4 to 0.8 parts by-weight per 100 parts by weight gelatin (dry weight).

The surfactant used in the present invention preferably has a hydrophilic lipophilic balance (HLB) value of 38 to 42. A typical example of a suitable anionic surfactant is sodium lauryl sulphate which has an HLB value of 38 to 42. The surfactant is preferably used in a weight range of 0.05 to 0.1 parts by weight per 100 parts by weight gelatin (dry weight).

The gelatin/diacetylated monoglyceride/surfactant mixture used for gelatin capsule manufacture according to the invention may include further optional components, e.g. coloring agents, and other conventional capsule shell components. All components in the mixture are preferably'physiologically tolerable. For capsule manufacture, the viscosity of the mixture may be selected in conventional fashion so as to result in production of gelatin films of the desired thickness. Typically the gelatin content is about 25 to 35% w/v.

Before capsule manufacture, the gelatin mixture should desirably be allowed to stand, e.g. for a period of hours, for example 3 to 10 hours, to allow any bubbles to disappear.

The capsules according to the invention may be empty (e.g. as paired but non-fused body and cap components or as separate body and cap components) or they may be filled, e.g. with a pharmaceutical, vitamin, essential oil, or neutraceutical component or component mixture.

The following describes in general terms steps and methods for preparing a hardshell gelatin capsule with reduced static electricity and enhanced film lubrication according to the present invention.

In a first step, diacetylated monoglyceride is dispersed in purified water together with sodium lauryl sulfate as surfactant, e.g. in a weight ratio of 1 part diacetylated monoglyceride and surfactant to 2 to 3.5 parts water. The dispersion is stirred and homogenized to become an emulsion, e.g. using a stirring rate of 2,700-3,300 rpm for about 2 hours.

The emulsion is added to a gelatin solution. Typically this is produced by stirring gelatin and water (e.g. about 30% w/v gelatin) until the gelatin has completely dissolved.

Then, typically, a coloring agent, such as titanium dioxide is added to the gelatin solution and it is allowed to stand after the viscosity has been adjusted to the desired level. After complete removal of bubbles, the gelatin film is formed and the capsules are produced in a capsule manufacturing machine according to a conventional method.

Where the total content of diacetylated monoglycerides and surfactant is less than 0.3 parts by weight, their effect in reducing static electricity and enhancing film lubrication is not satisfactory. On the other hand, where the total content of diacetylated monoglycerides and surfactant is more than 1.0 parts by weight, the surface of the gelatin capsules becomes uneven.

The gelatin, diacetylated monoglycerides and sodium lauryl sulfate used in the Examples below have the properties described in The United States Pharmacopeia 24 and the US National Formulary 19.

### Example 1 - Capsule preparation

12.5 L of purified water at about 60°C is measured and placed in a vessel. With stirring the water at about 3,000 rpm, 0.5 kg of sodium lauryl sulfate is added.

Then, 4.0 kg of diacetylated monoglyceride is added and stirring is continued for 2 hours to produce an emulsion.

The emulsion is added to a gelatin solution (concentration 31.25 w/v%) to be in the proportion of 0.8 kg of diacetylated monoglyceride to 100 kg of gelatin. The gelatin solution is stirred at 60 rpm for about 2 hours until the completion of solubilization.

Then a coloring agent (e.g. titanium dioxide or another pigment) is added to gelatin solution and it is allowed to stand for more than 4 hours after adjusting viscosity. After complete removal of bubbles, gelatin film is formed into capsules in a capsule manufacturing machine according to a conventional method.

Hardshell gelatin capsules used as controls in the following Examples were manufactured by the conventional method using a gelatin solution but without adding an emulsion containing diacetylated monoglyceride and sodium lauryl sulfate.

### Example 2 - Static electricity occurrence test

To measure the static electricity occurrence, a cylindrical drum (Φ: 445 mm) having a rectangular outlet (210 mm x 230 mm) on the bottom side was used. On the opposite side, a 700 mm scale was attached.

The measuring method was as follows. After sealing the outlet, about 90,000 capsules are placed in the drum. This drum is placed on a 1,000 mm high support,- and the outlet is opened allowing the capsules to drop down. The static electricity is measured by a Static V. Meter "STATIRON-M" [made by Shisido Co., Ltd. Japan] at 30 mm distance from the outlet.

Table 1 shows the result for the static electricity occurrence test comparing the capsules of the invention and the control capsules. As shown in Table 1, the occurrence of static electricity with the capsules of the invention is remarkably reduced.

**Table 1**

| **Comparison of occurrence of static electricity** | | |
|---|---|---|
| | Capsules of invention | Control capsules |
| Static electricity occurrence (kV) | 3-4 | 7-8 |

### Example 3 - Lubrication test

The measuring method is as follows. After sealing the outlet of the drum described in Example 2, about 90,000 capsules are placed in the drum. This drum is placed on a 1,000 mm high support, and the outlet is opened allowing the capsules to drop down. After the capsules have all dropped, the height of the pile of dropped capsules is measured. The lower the height the better the capsule surface lubrication.

Table 2 shows the result for the lubrication test (sliding test) comparing the capsules according to the present invention and the control capsules. As shown in Table 2, the lubrication of the capsules of the invention is remarkably enhanced.

**Table 2**

| **Comparison of lubrication** | | |
|---|---|---|
| | Capsules of invention | Control capsules |
| Sliding test | 19 cm | 26 cm |

### Example 3 - Filling property and Quality tests

Table 3 shows the result of filling property testing comparing the capsules of the present invention and the control capsules. As shown in Table 3, the filling property of the capsules of the invention is excellent and there were no observed joint defects, such as bad joint and telescope in filling.

**Table 3**

| **Comparison of filling property** | | | | | | |
|---|---|---|---|---|---|---|
| | Zanasi AZ-20 | | | Zanasi AZ-40 | | |
| Filing speed | 15,000 /hour | | | 35,000 /hour | | |
| Pressure | 26664.4 Pa (20 cmHg) | | | 33330.5 Pa (25 cmHg) | | |
| Filing Q'ty | 100,000 | | | 100,000 | | |
| Result | | Present invention capsules | Control capsules | | Present invention capsules | Control capsules |
| | Water content | 14.0% | 14.0% | Water content | 13.5% | 13.5% |
| | Joint defects | none | 12 | Joint defects | none | 20 |

Table 4 shows the result of a quality test comparing the capsules of the present invention and the control capsules which involved measuring bad joint and telescope. As shown in Table 4, the quality of the capsules of the invention is remarkably enhanced in terms of reduction in bad joint and telescope, which are defects sometimes observed in capsule manufacturing and filling processes.

**Table 4**

| **Comparison of quality** | | | | |
|---|---|---|---|---|
| | Present invention capsules | | Control capsule | |
| Speed of machine | 70,000 /hour | | 70,000 /hour | |
| Spec of capsule | Size : #1 | | Size : #1 | |
| Quantity | 4,500,000 caps | | 4,500,000 caps | |
| Result | Bad joint | Telescope | Bad joint | Telescope |
| | 1 | none | 50 | 8 |

### Example 5 - Solubility and Disintegration tests

For solubility testing, measuring was carried out according to the Japanese Pharmacopeia. 50 ml of purified water is placed in a 100 ml flask. At 37 ± 0.5°C, the time required for completely dissolving the capsule after separating cap and body components is measured. 5 measurements are made for each group of capsules. The standard requirement is that the capsules dissolve within 10 minutes.

For disintegration testing, measuring was carried out according to the Japanese Pharmacopeia. The time required for complete disintegration of the capsules at 37 ± 0.5°C is measured. 5 measurements are made for each group of capsules. The standard requirement is that the capsules disintegrate within 20 minutes.

Table 5 shows the results of the solubility and disintegration tests comparing the capsules of the present invention and the control capsules.

As shown in Table 5, the solubility and disintegration properties of the capsules of the invention are almost equal to those of the control capsules. Both meet the standard requirements in the Japanese Pharmacopeia.

**Table 5**

| **Comparison of solubility and disintegration** | | |
|---|---|---|
| | Present invention capsules | Control capsules |
| Solubility | Avg. 3'16" | Avg. 3'10" |
| (pH 6.0-7.0) | Max. 3'24" | Max. 3'20" |
| | Min. 3'08" | Min. 3'00" |
| Disintegration | Avg. 12'20" | Avg. 12'13" |
| (pH 6.0-7.0) | Max. 12'40" | Max. 12'35" |
| | Min. 12'00" | Min. 11'50" |

As described above, the present invention provides an efficient gelatin capsule with reduced static electricity and enhanced film lubrication. Further, the present invention enables increased capsule productivity by improving each of the manufacturing steps: capsule transfer step; capsule printing step; and capsule filling step.

## Claims

1. A process for the preparation of gelatin capsules, said process comprising:
i) forming an emulsion containing diacetylated monoglyceride and sodium lauryl sulphate;
ii) mixing said emulsion and a gelatin solution in a weight ratio of 0.3 to 1.0 parts diacetylated monoglyceride and surfactant to 100 parts gelatin; and
iii) forming said capsules.

2. A process as claimed in claim 1 which comprises the steps of:
i) preparing an emulsion containing 0.3-1.0 parts by weight of diacetylated monoglycerides and sodium lauryl sulfate;
ii) adding said emulsion to a gelatin solution containing 100 parts by weight of gelatin to form a mixed solution;
iii) mixing and homogenizing said mixed solution; and
iv) adjusting viscosity, standing for a a period of hours to allow any bubbles to disappear and forming a capsule.

3. A process as claimed in either of claims 1 and 2 wherein the diacetylated monoglyceride used is a transparent liquid.

4. A process as claimed in any one of claims 1 to 3 wherein sodium lauryl sulfate having an HLB value of 38 to 42 is used in said emulsion.

5. A process as claimed in any one of claims 1 to 4 wherein the diacetylated monoglyceride is mixed with the gelatin in a weight ratio of 0.4:100 to 0.8:100.

6. A process as claimed in any one of claims 1 to 5 wherein sodium lauryl sulfate is mixed with the gelatin in a weight ratio of 0.05:100 to 0.1:100.

7. A gelatin capsule or capsule component produced by a process as claimed in any one of claims 1 to 6.

8. The use of a diacetylated monoglyceride and a surfactant in the manufacture of gelatin capsules and gelatin capsule components.

9. A gelatin capsule, the gelatin shell whereof comprises diacetylated monoglyceride, surfactant and gelatin in a weight ratio of diacetylated monoglyceride and surfactant to gelatin of 0.3:100 to 1.0:100.

## Patentansprüche

1. Verfahren zur Herstellung von Gelatinekapseln, bei dem man:
i) eine Emulsion herstellt, die diacetyliertes Monoglycerid und Natriumlaurylsulfat enthält;
ii) die Emulsion und eine Gelatinelösung in einem Gewichtsverhältnis von 0,3 bis 1,0 Teile diacetyliertes Monoglycerid und oberflächenaktives Mittel auf 100 Teile Gelatine mischt; und
iii) die Kapseln formt.

2. Verfahren nach Anspruch 1, bei dem man:
i) eine Emulsion herstellt, die 0,3 bis 1,0 Gewichtsteile diacetylierte Monoglyceride und Natriumlaurylsulfat enthält;
ii) die Emulsion zu einer Gelatinelösung hinzufügt, die 100 Gewichtsteile Gelatine enthält, wobei man eine gemischte Lösung erhält;
iii) die gemischte Lösung mischt und homogenisiert; und
iv) die Viskosität einstellt, über eine Dauer von mehreren Stunden stehen lässt, damit alle Luftblasen entweichen, und eine Kapsel formt.

3. Verfahren nach Anspruch 1 oder 2, wobei das eingesetzte diacetylierte Monoglycerid eine durchsichtige Flüssigkeit ist.

4. Verfahren nach einem der Ansprüche 1 bis 3, wobei man in der Emulsion Natriumlaurylsulfat mit einem HLB-Wert von 38 bis 42 verwendet.

5. Verfahren nach einem der Ansprüche 1 bis 4, wobei man das diacetylierte Monoglycerid mit der Gelatine in einem Gewichtsverhältnis von 0,4 : 100 bis 0,8 : 100 mischt.

6. Verfahren nach einem der Ansprüche 1 bis 5, wobei man das Natriumlaurylsulfat mit der Gelatine in ein Gewichtsverhältnis von 0,05 : 100 bis 0,1 : 100 mischt.

7. Gelatinekapsel oder -kapselbestandteil, hergestellt nach einem Verfahren nach einem der Ansprüche 1 bis 6.

8. Verwendung eines diacetylierten Monoglycerids und eines oberflächenaktiven Mittels zur Herstellung von Gelatinekapseln und Gelatinekapselbestandteilen.

9. Gelatinekapsel, deren Gelatinehülle ein diacetyliertes Monoglycerid, oberflächenaktives Mittel und Gelatine in einem Gewichtsverhältnis von diacetyliertem Monoglycerid und oberflächenaktivem Mittel zu Gelatine von 0,3 : 100 bis 1,0 : 100 umfasst.

## Revendications

1. Procéde de préparation de gélules en gélatine, ledit procédé comprenant :
i) la formation d'une émulsion contenant un monoglycéride diacétylé et du laurylsulfate de sodium ;
ii) le mélange de ladite émulsion et d'une solution de gélatine selon un rapport en poids de 0,3 à 1,0 partie du monoglycéride diacétylé et du tensioactif à 100 parties de gélatine ; et
iii) la formation desdites gélules.

2. Procédé selon la revendication 1, qui comprend les étapes de :
i) préparation d'une émulsion contenant 0,3-1,0 partie en poids de monoglycérides diacétylés et de laurylsulfate de sodium ;
ii) addition de ladite émulsion à une solution de gélatine contenant 100 parties en poids de gélatine pour former une solution mixte ;
iii) mélange et homogénéisation de ladite solution mixte ; et
iv) ajustement de la viscosité, abandon pendant un laps de temps de quelques heures pour permettre la disparition de toutes bulle, et formation d'une gélule.

3. Procédé selon l'une ou l'autre des revendications 1 et 2, dans lequel le monoglycéride diacétylé utilisé est un liquide transparent.

4. Procédé selon l'une quelconque des revendications 1 à 3, dans lequel on utilise dans ladite émulsion un laurylsulfate de sodium ayant un indice HLB de 38 à 42.

5. Procédé selon l'une quelconque des revendications 1 à 4, dans lequel le monoglycéride diacétylé est mélangé à la gélatine selon un rapport en poids de 0,4:100 à 0,8:100.

6. Procédé selon l'une quelconque des revendications 1 à 5, dans lequel le laurylsulfate de sodium est mélangé à la gélatine selon un rapport en poids de 0,05:100 à 0,1:100.

7. Gélule de gélatine ou composant de gélule de gélatine produit par un procédé selon l'une quelconque des revendications 1 à 6.

8. Utilisation d'un monoglycéride diacétylé et d'un tensioactif pour la fabrication de gélules de gélatine et de composants de gélule de gélatine.

9. Gélule de gélatine, dont la gaine de gélatine comprend un monoglycéride diacétylé, un tensioactif et de la gélatine selon un rapport en poids du monoglycéride diacétylé et du tensioactif à la gélatine de 0,3:100 à 1,0:100.
